(19) Europäisches Patentamt
European Patent Office
Office européen des brevets

(11) **EP 4 151 151 A1**

(12) **EUROPEAN PATENT APPLICATION**

(43) Date of publication:
**22.03.2023 Bulletin 2023/12**

(21) Application number: **21197921.6**

(22) Date of filing: **21.09.2021**

(51) International Patent Classification (IPC):
**A61B 5/1473** (2006.01)     **A61B 5/1486** (2006.01)

(52) Cooperative Patent Classification (CPC):
**A61B 5/14532; A61B 5/1473;** A61B 5/14735;
A61B 5/14865; A61B 2562/0209; A61B 2562/125

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **F. Hoffmann-La Roche AG**
**4070 Basel (CH)**
Designated Contracting States:
**AL AT BE BG CH CY CZ DK EE ES FI FR GB GR
HR HU IE IS IT LI LT LU LV MC MK MT NL NO PL
PT RO RS SE SI SK SM TR**
• **Roche Diabetes Care GmbH**
**68305 Mannheim (DE)**
Designated Contracting States:
**DE**

(72) Inventor: **KEUBLER, Sebastian**
**68305 Mannheim (DE)**

(74) Representative: **Freiherr von Campenhausen,
Harald
Roche Diagnostics GmbH
Patentabteilung
Sandhofer Straße 116
68305 Mannheim (DE)**

(54) **SENSOR WITH VARYING STIFFNESS**

(57)     The present invention relates to an analyte sensor comprising a substrate, a first conductive material, a second conductive material, a first layer and a second layer, wherein the first and the second layer, independently of one another have a varying thickness along the length of the substrate and/or are located on the substrate as at least two fields separate from one another. The present invention furthermore relates to a method for manufacturing the analyte sensor and an analyte sensor system comprising the analyte sensor.

Fig. 1

**Description**

Technical Field

The present invention relates to an analyte sensor comprising a substrate, a first conductive material, a second conductive material, a first layer and a second layer, wherein the first and the second layer, independently of one another have a varying thickness along the length of the substrate and/or are located on the substrate as at least two fields separate from one another. The present invention furthermore relates to a method for manufacturing the analyte sensor and an analyte sensor system comprising the analyte sensor. The analyte sensor according to the present invention may mainly be used for conducting analyte measurements in a body fluid of the user.

Background

Biosensors for measuring analytes in biological fluids, in particular a sensor which is designed for implantation or subcutaneous insertion to measure body fluids, have to fulfill a variety of functions: on the one hand, the sensor ideally provide for specific and sensitive analyte measurement without interference from e.g. particular components of body fluids. For this purpose, biosensors are frequently covered with membranes with at least reduced permeability to particular compounds in order to allow access to the actual sensing sites only for low molecular weight compounds. While the specificity of biosensors is achieved by using of bio recognition elements, such as enzymes, the sensitivity is often tailored by using of diffusion limiting membranes. Finally, the implanted sensor must be biocompatible to prevent an inflammatory reaction of the body against the sensor and for this purpose, an additional biocompatibility membrane may be applied.

Moreover, with implanted sensors, it is preferred to have sensors which can remain in place for a long period such as 3 to 21 days without deterioration of the measurement, in order to spare the patient frequently exchanging the sensor.

Implanted sensors, for example, comprise electrode systems which facilitate measurements of physiologically significant analytes such as, for example, like that of glucose in the patient's body. The working electrodes of such a sensor have electrically conductive enzyme layers in which enzyme molecules are bound which release charge carriers by catalytic conversion of the analyte molecules. In this process, an electrical current is generated as a measuring signal the amplitude of which correlates to the analyte concentration. These types of sensors are also called electrochemical sensors.

US 9,895,091 B2 discloses electrochemical sensors. These electrochemical sensors can comprise an impermeable dielectric layer on top of Ag/AgCl of a reference electrode. This coating is used to extend the reference electrode's lifetime. The electrochemical sensor disclosed has a layered structure wherein the reference electrode is positioned on top of a working electrode. The working electrode is separated from the reference electrode by an insulating layer.

WO 2006/017359 describes a sensor which comprises two wires. The wires may have a variable diameter and/or one wire may be wrapped around the other with varying pitch to obtain a sensor with varying stiffness along its length.

The manufacturing of the sensors described in the prior art is very time- and cost-consuming. Further they have drawbacks with regard to their wearing comfort and mechanical stability during wear time.

Problem to be solved

It is therefore an object of the present invention to provide an analyte sensor which avoids at least in part certain drawbacks of the prior art, in particular with regard to its manufacturability, their wearing comfort and their mechanical stability during wear time.

Summary of the invention

This problem is solved by the analyte sensor according to independent claim 1 as well as by the method for manufacturing this sensor according to independent claim 14 and by the analyte sensor system according to independent claim 15. Preferred embodiments of the invention which may be realized in an isolated way or in any arbitrary combination are disclosed in the dependent claims and throughout the specification.

The inventive analyte sensor is particularly easy to manufacture as essentially all layers, in particular the first layer and the second layer, comprised in the analyte sensor can be applied to the substrate by known techniques, such as by spin-coating, spray-coating, doctor-blading, printing, dispensing, slot-coating, dip-coating and/or screen-printing. Furthermore, the stiffness and flexibility of the analyte sensor along its length can be targeted to specific needs by the specific location of the first layer relative to the second layer. This results in a particularly good mechanical stability of the analyte sensor during wear time and to a good wearing comfort for the user of the analyte sensor. Furthermore, it reduces the requirements for the insertion process as well as for the adhesion of the plaster over the wear time. As the analyte sensor has a higher flexibility, the risk that the analyte sensor is damaged during insertion and/or due to a plaster which has less adhesion, is reduced. Furthermore, in particular if the analyte sensor behaves like a spring as described below, the robustness of the analyte sensor is increased and the number of malfunctions can be reduced as the analyte sensor has a higher robustness against external impact so that bending of the analyte sensor does not influence the measurement or influences the measurement only to a reduced extent.

As used in the following, the terms "have", "comprise", or "include" or any arbitrary grammatical variations thereof are used in an exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i. e. a situation in which A solely and exclusively consists of B) and to a situation in which, besides B, one or more further elements are present in entity A, such as element C, elements C and D or even further elements.

Further, it should be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, notwithstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restrictions regarding alternative embodiments of the invention, without any restrictions regarding the scope of the invention and without any restrictions regarding the possibility of combining the features introduced in such way with the optional or nonfunctional features of the invention.

In a first aspect of the present invention an analyte sensor for determining at least one analyte, the analyte sensor comprising:

- a substrate which has a length and a width and which comprises

  • a first side and a second side which opposes the first side,
  • an in-vivo portion, an ex-vivo portion and an intermediate portion wherein the intermediate portion connects the in-vivo portion with the ex-vivo portion,

- at least one first conductive material which is located on the first side of the substrate,
- at least one second conductive material which is located on the second side of the substrate,
- at least one first layer which is located on the first side of the substrate and covers the first conductive material partially and

  • which has a varying thickness along the length of the substrate and/or
  • which is located on the first side of the substrate as at least two fields of the first layer which are separate from one another wherein each of the at least two fields extends over the whole width of the substrate,

- at least one second layer which is located on the second side of the substrate and covers the second conductive material partially and

  • which has a varying thickness along the length of the substrate and/or
  • which is located on the second side of the substrate as at least two fields of the second layer which are separate from one another wherein each of the at least two fields extends over the whole width of the substrate

is disclosed.

The term "analyte sensor" within the context of the present invention may refer to any device configured for the detection of an analyte.

The term "analyte" may refer to any arbitrary element, component or compound which may be present in a body fluid and the concentration of which may be of interest for the user. Preferably, the analyte may be or may comprise an arbitrary chemical substance or chemical compound which may take part in the metabolism of the user, such as at least one metabolite. As an example, the analyte may be selected from the group consisting of glucose, ketone, cholesterol, triglycerides, and lactate. Additionally or alternatively, however, other types of analytes and/or any combination of analytes may be determined. Preferably, the analyte is glucose.

Thus, the analyte sensor is preferably a biosensor. Further, preferably, the analyte sensor is an electrochemical sensor. The term "electrochemical sensor" refers to a sensor which is adapted for performing at least one electrochemical measurement, in particular, a plurality or series of electrochemical measurements, in order to detect the analyte comprised within the body fluid by using an amperometric method. Especially, the term "electrochemical measurement" refers to the detection of an electrochemically detectable property of the analyte, such as an electrochemical detection reaction, by employing amperometric methods. Thus, for example, the electrochemical detection may be carried out by applying and comparing one or more electrical potentials. Specifically, the electrochemical sensor may be adapted to generate at least one electrical measurement signal which may directly or indirectly indicate the presence and/or absence of the electrochemical detection reaction, such as at least one current signal and/or at least one voltage signal. The measurement may be a quantitative and/or a qualitative measurement.

The analyte sensor is partially implantable and may, thus, be adapted for performing the detection of the analyte in the body fluid in the subcutaneous tissue, in particular, an interstitial fluid. As used herein the terms "implantable" or "subcutaneous" refer to be fully or at least partially arranged within the body tissue of the user which may be a human or animal, preferably partially arranged within the body tissue of the user. For this purpose, the analyte sensor comprises an insertable portion, the in-vivo portion, wherein the term "insertable portion" may generally refer to a part or component of an element configured to be insertable into an arbitrary body tissue while other parts or components, such as the ex-vivo portion, may remain outside of the body tissue. Preferably, the body tissue is skin. Preferably, the insertable portion may fully or partially comprise a biocompatible membrane, i. e. a surface which may have as little detrimental effects on the user, the patient, or the body tissue as possible, at least during typical durations of use.

Thus, preferably, the analyte sensor of the present invention is an implantable sensor.

As generally used, the term "body fluid" may refer to fluid, in particular liquid, which may typically be present in a body or a body tissue of the user or the patient and/or which may be produced by the body of the user or the patient. Preferably, the body fluid may be selected from the group consisting of blood and interstitial fluid. However, additionally or alternatively, one or more other types of body fluid may be used, such as saliva, tear fluid, urine or other body fluids. During the detection of the analyte, the body fluid may be present within the body or body tissue. Thus, the analyte sensor may be configured for detecting the analyte within the body tissue. The analyte sensor is in one embodiment suitable for short-term application, e. g. 3 to 21 days, or for long-term application e. g. 1 to 12 months. During its application, the analyte may be determined by continuous or discontinuous measurements.

The inventive analyte sensor comprises a substrate which has a length and a width. It comprises a first side and a second side which opposes the first side and it comprises an in-vivo portion, and ex-vivo portion and an intermediate portion wherein the intermediate portion connects the in-vivo portion with the ex vivo portion.

Within the context of the present invention, the term "substrate" specifically may refer, without limitation, to any kind of material or combination of materials which is suitable to form a carrier layer to support the at least one first conductive material and the at least one second conductive material. In particular, the substrate may comprise an electrically insulating material. Within the context of the present invention "electrically insulating material" is a broad term and given its ordinary and customary meaning to a person of ordinary skill in the art. The term "electrically insulating material" may also encompass a dielectric material. The term specifically may refer, without limitation, to a material or combination of materials which prevent the transfer of electrical charges and which do not sustain a significant electrical current. Specifically, without limiting other possibilities, the at least one electrically insulating material may be or may comprise at least one insulating resin such as insulating epoxy resins used in manufacturing of electronic printed circuit boards. In particular, it may comprise or be at least one thermoplastic material such as a polycarbonate, a polyester, a polyvinylchloride, a polyurethane, a polyethylene, a polypropylene, polystyrene, a polyether, a polyamide, a polyimide, polytetrafluoroethylene or a copolymer thereof. In an embodiment, the at least one electrically insulating material may comprise or may be alumina. Suitable polyesters are, for example, selected from the group consisting of polyethylene terephthalate (PET), glycol modified polyethylene terephthalate, and polyethylene naphthalate. A suitable polyethylene is for example selected from the group consisting of high density polyethylene (HDPE) and low density polyethylene (LDPE).

Thus in a preferred embodiment the substrate comprises at least one electrically insulating material selected from the group consisting of an insulating epoxy resin, a polycarbonate, a polyester, a polyvinylchloride, a polyurethane, a polyethylene, a polypropylene, polystyrene, a polyether, a polyamide, a polyimide, polytetrafluoroethylene or a copolymer thereof, and alumina.

The substrate comprises a first side and a second side which opposes the first side. Thus, in particular, the substrate comprises two opposing sides, the first side and the second side. As generally used the term "side" refers to a surface of the substrate. Herein, the terms "first" and "second" are considered as description without specifying an order and without excluding an embodiment in which other elements of the same kind may be present.

The substrate may be a flat substrate. The substrate may have an elongated shape, such as a strip shape or a bar shape. As generally used, the term "elongated shape" indicates that each surface of the planar body has an extension in a direction along the elongation which exceeds an extension perpendicular hereto by at least a factor of 2, at least a factor of 5, at least a factor of 10 or even at least a factor of 20 or more. The direction of the substrate which has the largest extension is usually referred to as "length of the substrate". The smallest direction which is perpendicular to the length of the substrate is typically referred to as "thickness of the substrate". The direction which is perpendicular to both, the length of the substrate and the thickness of the substrate is typically referred to as "width of the substrate". The substrate has a width and a length.

Specifically the substrate may be flexible and/or deformable. In particular, the substrate may be bendable. Thus, as an example, the substrate may be a thin, flexible substrate. As an example, the substrate may have a

thickness of 50 μm to 1 mm, specifically a thickness of 80 μm to 500 μm, such as 110 μm to 250 μm.

The substrate may have a length which is preferably less than 50 mm, such as a length of 30 mm or less, e.g. a length of 5 mm to 30 mm.

The length of the substrate is in particular measured in the insertion direction of the analyte sensor. The length of the substrate refers to the total length of the substrate. The "total length of the substrate" is the overall length of the substrate, including the in-vivo portion, the ex-vivo portion and the intermediate portion.

The substrate typically has a width in the range from 100 μm to 1 mm, preferably in the range from 300 μm to 800 μm.

The substrate comprises an in-vivo portion, an ex-vivo portion and an intermediate portion. The intermediate portion connects the in-vivo portion with the ex-vivo portion. The in-vivo portion of the substrate is typically the part of the substrate which may be arranged within the body tissue of the user. It is typically also referred to as insertable portion. The ex-vivo portion refers is typically the part of the substrate which may be arranged outside the body of the user. The ex-vivo portion of the substrate typically comprises means, such as electrical contacts for connecting the analyte sensor with an electronics unit. The intermediate portion connects the in-vivo portion with the ex vivo portion. The intermediate portion may be fully or partially arranged within the body tissue of the user. It may additionally or alternatively be fully or partially arranged outside the body of the user.

The analyte sensor comprises at least one first conductive material which is located on the first side of the substrate.

The analyte sensor furthermore comprises at least one second conductive material which is located on the second side of the substrate.

The at least one first conductive material and the at least one second conductive material are preferably capable of sustaining electrical current. Thus, the at least one first conductive material is in particular at least one first electrically conductive material. The at least one second conductive material is in particular at least one second electrically conductive material.

"Electrically conductive material" within the context of the present invention refers to a material being capable of sustaining an electrical current. Thus, an electrically conductive material may be selected from the group consisting of metals, and nonmetallic electrically conductive materials.

Suitable metals are known as such and are, for example, selected from the group consisting of gold, nickel, platinum, and palladium, wherein gold is particularly preferred. Also gold paste is suitable as electrically conductive material.

Suitable nonmetallic electrically conductive materials are for example selected from the group consisting of carbon, carbon paste, doped metal oxides, such as fluorine doped tin oxide (FTO) and indium doped tin oxide (ITO), or conductive polymers. Suitable conductive polymers are, for example polyaniline and/or poly-3,4-ethylenedioxythiophene (PEDOT). Carbon paste may comprise, for example, carbon and a solvent such as diethylene glycol butyl ether and at least one binder such as vinyl chloride co- and terpolymers. Carbon paste is known as such.

Thus, the at least one electrically conductive material preferably is selected from the group consisting of gold, nickel, platinum, palladium, carbon, carbon paste, polyaniline and poly-3,4-ethylenedioxy-thiophene (PEDOT), particularly preferred, the at least one electrically conductive material is selected from the group consisting of gold, carbon, and carbon paste. More preferably, the at least one electrically conductive material consists essentially of gold and/or carbon and/or carbon paste. In an embodiment, the at least one electrically conductive material has a layered structure wherein a first layer consists of gold and a second layer consists of carbon and/or carbon paste. In this embodiment, preferably, gold is positioned on top of the first side of the substrate and on top of the gold, carbon and/or carbon paste is positioned.

The embodiments and preferences described for the at least one electrically conductive material apply independently of one another for the at least one first electrically conductive material and for the at least one second electrically conductive material.

The substrate may comprise the at least one first conductive material, in particular the at least one first electrically conductive material in the form of at least one first conductive trace. The substrate may comprise the at least one second conductive material, in particular the at least one second electrically conductive material in the form of at least one second conductive trace.

The term "conductive trace" within the context of the present invention refers, without limitations, to an electrically conductive strip, layer, wire or other type of electrical conductor. The conductive trace may have a thickness of at least 0.05 μm, preferably of at least 0.5 μm, more preferably of at least 5 μm, specifically of at least 7 μm, or at least 10 μm. In the case where the conductive trace comprises carbon or is carbon, the conductive trace may specifically have a thickness of at least 7 μm, more specifically of at least 10 μm. Specifically, in the case where the conductive trace is gold, the conductive trace may have a thickness of at least 50 nm, more specifically of at least 100 nm.

The at least one electrically conductive material may be positioned on the substrate by any known method, for example via chemical vapor deposition (CVD), physical vapor deposition (PVD), or a wet-coating process. Wet-coating processes are known as such. A suitable wet-coating process is for example selected from the group consisting of spin-coating, spray-coating, doctor-blading, printing, dispensing, slot-coating, dip coating and screen printing.

The analyte sensor comprises a first layer which is located on the first side of the substrate and covers the first conductive material partially.

The first layer may comprise any material suitable for use in an analyte sensor. The first layer may consist of any material suitable for use in an analyte sensor. In particular, the first layer may comprise at least one material selected from the group consisting of a third conductive material, an insulating material, a sensing material, a biocompatibility material and a flux limiting material.

Thus, an analyte sensor is preferred in which the first layer comprises at least one material selected from the group consisting of a third conductive material, an insulating material, a sensing material, a biocompatibility material and a flux limiting material.

A suitable third conductive material is preferably capable of sustaining electrical current. Thus, the at least one third conductive material is in particular at least one third electrically conductive material. For the third electrically conductive material, the embodiments and preferences described above for the first electrically conductive material and the second electrically conductive material apply.

The third conductive material may be the same as the first conductive material and/or the second conductive material. It is also possible and in an embodiment of the present invention even preferred that the third conductive material is different from the first conductive material and/or the second conductive material.

The third electrically conductive material may be selected from the group consisting of metals, and nonmetallic electrically conductive materials.

Suitable metals are known as such and are, for example, selected from the group consisting of gold, nickel, platinum, and palladium, wherein gold is particularly preferred. Also gold paste is suitable as electrically conductive material.

Suitable nonmetallic electrically conductive materials are for example selected from the group consisting of carbon, carbon paste, doped metal oxides, such as fluorine doped tin oxide (FTO) and indium doped tin oxide (ITO), or conductive polymers. Suitable conductive polymers are, for example polyaniline and/or poly-3,4-ethylenedioxythiophene (PEDOT). Carbon paste may comprise, for example, carbon and a solvent such as diethylene glycol butyl ether and at least one binder such as vinyl chloride co- and terpolymers. Carbon paste is known as such.

Thus, the at least one third electrically conductive material preferably is selected from the group consisting of gold, nickel, platinum, palladium, carbon, carbon paste, polyaniline and poly-3,4-ethylenedioxythiophene (PEDOT), particularly preferred, the at least one third electrically conductive material is selected from the group consisting of gold, carbon, and carbon paste. More preferably, the at least one third electrically conductive material consists essentially of gold and/or carbon and/or carbon paste.

In an embodiment, it is preferred that the first conductive material comprises or consists of gold and/or carbon and/or carbon paste and the third conductive material comprises or consists of carbon and/or carbon paste.

If the first layer is or comprises at least one third conductive material, then the first layer is preferably positioned on the in-vivo portion of the substrate.

A suitable insulating material may be any material which insulates, in particular which electrically insulates. Specifically, the insulating material may comprise at least one organic, electrically insulating material and/or at least one inorganic, electrically insulating material. In particular, the insulating material may be selected from resins and resists, such as a solder resist.

Typically, the insulating material may be comprised in the first layer. It is also possible that the insulating material is comprised in the analyte sensor but does not form a first layer. The insulating material then typically forms an insulating layer which partially covers the first conductive material. In general, the insulating material, independent if it forms a first layer or if it forms an insulating layer is at least comprised on the ex-vivo portion of the substrate and on the intermediate portion of the substrate. Typically, the insulating material leaves a portion of the first conductive material on the ex-vivo portion of the analyte sensor open. This portion of the first conductive material then serves as electrical contact to contact the analyte sensor with an electronics unit, such as a printed circuit board. Typically also a portion of the in-vivo portion of the analyte sensor is covered by the insulating material.

A suitable sensing material may be any material which is sensitive for the analyte. Thus the sensing material preferably comprises at least one enzyme. The sensing material may comprise only one enzyme or a mixture of two or more enzymes. Only one enzyme is preferred. Specifically, the enzyme is capable of catalyzing a chemical reaction converting the analyte, in particular glucose. Even more specifically the at least one enzyme is selected from the group consisting of a glucose oxidase (EC 1.1.3.4), a hexose oxidase (EC 1.1.3.5), an (S)-2 hydroxy acid oxidase (EC 1.1.3.15), a cholesterol oxidase (EC 1.1.3.6), a glucose dehydrogenase, a galactose oxidase (EC 1.1.3.9), an alcohol oxidase (EC 1.1.3.13), an L-glutamate oxidase (EC 1.4.3.11), and an L-aspartate oxidase (EC 1.4.3.16). In particular, the at least one enzyme is a glucose oxidase (GOx) and/or modifications thereof.

If the first layer is or comprises a sensing material, then the sensing material together with the first conductive material forms at least one first electrode.

The sensing material may be applied by any known method to the at least one first conductive material, for example by a wet-coating process. A suitable wet-coating process is for example selected from the group consisting of spin-coating, spray-coating, doctor-blading, printing, dispensing, slot-coating, dip coating and screen printing. After the wet-coating process, the layer of the sensing material may be further treated. Such treatments are for example drying treatment, curing treatments and/or laser ablation treatments. Such treatments are known as such.

The term "sensing material", as used herein, specifically may refer, without limitation, to a material that may be or may comprise at least a polymeric material; specifically it may be or may comprise at least a polymeric material and at least a metal containing complex. The metal containing complex may be selected from the group consisting of transition metal element complexes, specifically the metal containing complex may be selected from osmium-complexes, ruthenium-complexes, vanadium-complexes, cobalt-complexes, and iron-complexes, such as ferrocenes, such as 2-aminoethylferrocene. Even more specifically, the sensing material may be a polymeric transition metal complex as described for example in WO 01/36660 A2, the content of which is included by reference. In particular, the sensing material may comprise a modified poly(vinylpyridine) backbone loaded with poly(bi-imidizyl) Os complexes covalently coupled through a bidentate linkage. A suitable sensing material is further described in Feldmann et al, Diabetes Technology & Therapeutics, 5 (5), 2003, 769-779, the content of which is included by reference. Suitable sensing materials further may include ferrocene-containing polyacrylamide-based viologen-modified redox polymer, pyrrole-2,2'-azino-bis(3-ethylbenzthiazoline-6-sulfonic acid)(ABTS)-pyrene, Naphthoquinone-LPEI. The polymeric transition metal complex may represent a redox mediator incorporated into a cross-linked redox polymer network. This is advantageous as it may facilitate electron transfer between the at least one enzyme or analyte and the conductive trace. In order to avoid a sensor drift, the redox mediator and the enzyme may be covalently incorporated into a polymeric structure.

In an embodiment the sensing material may comprise a polymeric material and $MnO_2$-particles or any other material catalyzing hydrogen peroxide oxidation reaction as well as the at least one enzyme. Another material catalyzing hydrogen peroxide oxidation reaction is Pt (platinum).

Moreover, the sensing material may additionally comprise at least one crosslinker; the crosslinker may for example be capable of crosslinking at least part of the sensing material. Specifically the sensing material may comprise at least one crosslinker selected from UV-curable crosslinkers and chemical crosslinkers; more specifically the sensing material comprises a chemical crosslinker. Alternatively, the sensing material may be free of any crosslinker. "Free of any crosslinker" as used herein, specifically may refer to a concentration of crosslinker in the range from 0 to 0.5 wt-% based on the dry weight of the sensing material. The term "dry weight" as used herein refers to the dry matter of the respective material, e.g. the material without the addition of any water or other solvent.

Suitable chemical crosslinkers according to the present invention are preferably selected from the group consisting of epoxide based crosslinkers, such as diglycidyl ethers like poly(ethylene glycol) diglycidyl ether (PEG-DGE) and poly(propylene glycol) diglycidyl ether; trifunctional short chain epoxides; anhydrides; diglycidyl ethers such as Resorcinol diglycidyl ether, Bisphenol A diglycidyl ether, Diglycidyl 1,2-cyclohexanedicarboxylate, Poly(ethylene glycol) diglycidyl ether, Glycerol diglycidyl ether, 1,4-Butanediol diglycidyl ether, Poly(propylene glycol) diglycidyl ether, Bisphenol diglycidyl ether, Poly(dimethylsiloxane), diglycidyl ether, Neopentyl glycol diglycidyl ether, 1,2,7,8-Diepoxyoctane, 1,3-Glycidoxypropyl-1,1,3,3-Tetramethyldisioxane; triglycidyl ethers such as N,N-Diglycidyl-4-glycidyloxyaniline, Trimethylolpropane triglycidyl ether; and tetraglycidyl ethers such as Tetrakisepoxy cyclosiloxane, Pentaerythritol tetraglycidyl ether, tetraglycidyl-4,4'-methylenebisbenzenamine.

The term "chemical crosslinker" as used herein, specifically may refer, without limitation, to a crosslinker that is capable of initiating a chemical reaction generating a crosslinked molecular network and/ or a cross-linked polymer when exposed to heat. "Exposed to heat" may refer to being exposed to a temperature above 15°C, specifically to a temperature above 20 °C; more specifically to a temperature in the range from 20 °C to 50 °C and even more specifically to a temperature in the range from 20 °C to 25 °C. More specifically, chemical crosslinkers may initiate crosslinking of the sensing material when exposed to heat.

The term "UV-curable crosslinker" as used herein, specifically may refer, without limitation, to the ability of a chemical substance of initiating a photochemical reaction generating a crosslinked molecular network and/ or a crosslinked polymer when irradiated by light in the UV spectral range. More specifically, UV-curable crosslinkers may initiate crosslinking of the layer of the sensing material when irradiated by UV light.

Suitable UV curable crosslinkers according to the present invention include: benzophenone, diazirine and azide. Particularly suitable UV-curable crosslinkers are for example selected from the group consisting of, benzophenone comprising cross-linkers, poly(Di(2-hydroxy 3 aminobenzophenonepropylene) glycol), Dibenzophenone 1,2-cyclohexanedicarboxylate, Bis[2-(4-azidosalicylamido)ethyl] Disulfide, reaction products of the reaction of 4-aminobenzophenone with any one of the above for the chemical cross-linker described di-glycidyl cross-linkers, triglycidyl cross-linkers and tetraglycidyl cross-linkers, an example of such a reaction product is 2,4,6,8-Tetramethyl-2,4,6,8-tetrakis(2-hydroxy 3-aminpropylbenzophenone)-cyclotetrasiloxan, and reaction products of the reaction of 4-Benzoylbenzoic Acid N-Succinimidyl Ester with a diamin or a jeffamin.

If the first layer does not comprise or is not a sensing material, then a layer of sensing material typically is located on the first side of the substrate and covers the first conductive material partially, thereby forming at least one first electrode. For the sensing material which is not comprised in the first layer, the embodiments and preferences described above for the sensing material which the first layer may be or may comprise apply accordingly.

Thus, the analyte sensor of the invention typically comprises at least one first electrode. Thus the analyte sensor of the invention is preferably an electrochemical sensor. Preferably, the at least one first electrode is at least one working electrode. Therefore, an analyte sensor is preferred wherein the at least one first electrode is at least one working electrode.

The analyte sensor of the invention is preferably an electrochemical sensor comprising at least one working electrode and at least one second electrode. More preferably, the sensor is an amperometric electrochemical sensor comprising at least one working electrode and the at least one second electrode. The working electrode is sensitive for the analyte to be measured at a polarization voltage which may be applied between the at least one working electrode and the at least one second electrode and which may be regulated by a potentiostat. A measurement signal may be provided as an electric current between the at least one first electrode, in particular the at least one working electrode, and the at least one second electrode.

If the first layer is or comprises a sensing material, then the first layer is preferably positioned on the in-vivo portion of the substrate.

A biocompatibility material as used herein relates to a material comprising, preferably consisting of a biocompatible material. Preferably, the biocompatibility material may be not diffusion-limiting for the analyte as specified elsewhere herein. For example, the biocompatibility material may be not diffusion-limiting for small molecules having a molecular weight of less than 2.000 Da, in an embodiment less than 1.000 Da. For examples, the biocompatibility material may not comprise an added enzyme. For example, the biocompatibility material may not comprise an added polypeptide. As will be understood by the skilled person this does not exclude that enzyme or polypeptide molecules diffuse into the biocompatibility material from adjacent layers, tissues or body fluids.

The term biocompatibility material as used herein relates to a material suitable for use with living tissue or a living system by not being or being to a reduced extent toxic, injurious, or physiologically reactive and/or causing to a reduced extent or not causing immunological rejection.. In an embodiment, the biocompatibility material is a material not inducing a bodily response, e.g. an inert material or a material comprising chemical compounds preventing bodily responses from occurring in the vicinity of the biocompatibility layer. In another embodiment, the biocompatibility material is a material preventing cells from attaching to said biocompatibility material. The biocompatibility material may be or may comprise at least one material selected from the group consisting of methacrylate based polymers and copolymers, such as acrylamide-methacrylate based copolymers, biodegradable polysaccharides such as hyaluronic acid (HA), agarose, dextran and chitosan. Further biocompatibility materials are disclosed in WO 2019/166394 A1 and include non biodegradable synthetic hydrogels such as hydrogels prepared from the copolymerization of 2-hydroxyethyl methacrylate (HEMA), 2-hydroxypropyl methacrylate (HPMA), acrylamide (AAm), acrylic acid (AAc), N-isopropylacrylamide (NIPAm), and methoxyl poly(ethylene glycol) (PEG) monoacrylate (mPEGMA or PEGMA), with cross-linkers, such as N,N'-methylenebis(acrylamide) (MBA), ethylene glycol diacrylate (EGDA) and PEG diacrylate (PEGDA), Pluronic® polymers with a structure of poly(ethylene oxide) (PEO)-poly(propylene oxide) (PPO)-PEO, chemical cross-linking of modified poly(vinyl alcohol) (PVA), Poly (4vinylpyridine), PEG.

A biocompatibility material may specifically be located positioned on top of the at least one first electrode. Preferably, it may also be located on top of a flux limiting material.

If the first layer is or comprises a biocompatibility material, then the first layer is preferably positioned at least on the in-vivo portion of the substrate.

A flux limiting material may, specifically refer to a material which provides a selective barrier. Thus, a flux limiting material generally may selectively allow for one or more molecules and/or compounds to pass, whereas its permeability for other molecules and/or compounds is significantly reduced. Thus, the flux limiting material may be permeable for the at least one analyte to be detected. As an example, the flux limiting material may be permeable for one or more of glucose, lactate, cholesterol or other types of analytes of the invention. The at least one flux limiting material may, hence function as a diffusion barrier that controls the diffusion of the analyte from the exterior, e.g. the body fluid surrounding the analyte sensor to the first electrode, preferably the sensing material, i.e. the at least one enzyme which may be comprised in the first electrode. In an embodiment, the flux limiting material may function as a biocompatibility material as mentioned elsewhere herein.

The at least one flux limiting material, as an example, may have a thickness sufficient for providing mechanical stability. As the at least one flux limiting material, as outlined herein, several materials may be used, standalone or in combination. Thus, as an example, the flux limiting material specifically may comprise at least one polymeric material. Suitable polymeric materials may, for example be selected from the group consisting of a polyvinylpyridine based copolymer, a polyurethane and a hydrogel. Polyvinylpyridine based copolymers are particularly suitable.

Suitable hydrogels are in particular polyethylene glycol copolymers (PEG copolymers), polyvinyl acetate copolymers (PVA copolymers), poly(2-alkyl-2-oxazonline) copolymers, polyacrylate and/or methacrylate-acrylate copolymers or block-copolymers, in particular polyacrylate and/or methacrylate-acrylate copolymers or block-copolymers comprising hydrophilic side groups. Thus, as an example, suitable hydrogels may be selected from the group consisting of (hydroxyethyl)methacrylate (HEMA) -homopolymers, HEMA-copolymers, silicon hydrogels and HEMA-co-N-vinylpyrrolidone-polymers, each of which may comprise side groups selected from the group consisting of methacrylic acid, glycerol methacrylate, N,N-dimethylacrylamide and phospharylcholine.

These types of flux limiting materials are generally known in the art. As an example, flux limiting materials as disclosed in e.g. EP2697388 A1, WO 2007/071562 A1 and/or in WO 2005/078424 A1 may be used. Specifically, the polymeric material may have a weight average molecular weight (MW) of more than 10.000 kDa. More specifically, the polymeric material may have a weight average molecular weight (MW) of more than 50.000 kDa, or even more than 100.000 kDa. Particularly suitable are polymeric materials with a weight average molecular weight (MW) of 10.000 to 500.000 kDa. The polymeric material of the flux limiting material may be the same as or may be different from the polymeric material of the sensing material.

If the first layer does not comprise or is not a flux limiting material, then a layer of flux limiting material typically is located on the first side of the substrate and covers the sensing material fully. For the flux limiting material which is not comprised in the first layer, the embodiments and preferences described above for the flux limiting material which the first layer may be or may comprise apply accordingly.

If the first layer is or comprises a flux limiting material, then the first layer is preferably positioned at least on the in-vivo portion of the substrate.

The analyte sensor comprises a second layer which is located on the second side of the substrate and which covers the second conductive material partially.

The second layer may comprise any material suitable for use in an analyte sensor. The second layer may consist of any material suitable for use in an analyte sensor. In particular, the second layer may comprise at least one material selected from the group consisting of a fourth conductive material, an insulating material, a silver containing material, a biocompatibility material, a flux limiting material and a hydrophobic polymer material.

Thus, an analyte sensor is preferred, in which the second layer comprises at least one material selected from the group consisting of a fourth conductive material, an insulating material, a silver containing material, a biocompatibility material, a flux limiting material and a hydrophobic polymer material.

For the fourth conductive material, the embodiments and preferences as described above for the third conductive material apply mutatis mutandis.

For the insulating material which may be comprised in the second layer the embodiments and preferences described above for the insulating material which may be comprised in the first layer apply mutatis mutandis.

For the biocompatibility material which may be comprised in the second layer the embodiments and preferences described above for the biocompatibility material which may be comprised in the first layer apply mutatis mutandis.

For the flux limiting material which may be comprised in the second layer the embodiments and preferences described above for the flux limiting material which may be comprised in the first layer apply mutatis mutandis.

A silver containing material may be any material which comprises silver. Thus, the silver containing material is also referred to as silver comprising material. "Silver" within the context of the present invention does not only encompass elemental silver, but also any silver comprising compound. Therefore, the silver containing material comprises elemental silver and/or at least one silver comprising compound. A preferred silver comprising compound is silver chloride (AgCl). For example, the silver containing material comprises elemental silver and/or silver chloride. In particular, the silver containing material comprises elemental silver and silver chloride. In particular, the silver containing material comprises silver / silver chloride (Ag/AgCl). In an embodiment, the silver containing material only comprises AgCl when the analyte sensor is manufactured. No elemental Ag is added when the analyte sensor is manufactured. During use of the analyte sensor, elemental Ag may then be formed from AgCl, so that during use, the analyte sensor comprises Ag/AgCl. The reaction of AgCl to form elemental Ag is known to the skilled person as such.

For example, the load of AgCl of the silver containing material is in the range from 20 $\mu$g to 150 $\mu$g. If at least two fields of the second layer are comprised in the analyte sensor and if the second layer comprises a silver containing material, in particular AgCl, then the load of AgCl of the silver containing material refers to the sum of the load of AgCl of the at least two fields. The load of AgCl of the silver containing material refers to the load when the analyte sensor is manufactured. It is clear to the skilled person that the load may change during use of the analyte sensor, for example due to the formation of elemental Ag from AgCl.

The minimum load of AgCl of the silver containing material may be calculated according to the following formula.

$$m(AgCl) = \frac{M(AgCl) * I * t}{z * F}$$

wherein

I        is the highest possible current in A while the analyte sensor is in use

t        is the total wear time of the sensor in s

F        is the Faraday constant in C/mol

z is the charge number of silver (z = 1)

M(AgCl) is the molar mass of AgCl

m(AgCl) is the load of AgCl of the at least one second electrode.

The silver containing material may comprise a binder. Suitable binders are known as such and are, for example, selected from the group consisting of metallic binders, ceramic binders and polymeric binders. Preferred are polymeric binders, in particular physically binding polymer binders and/or chemically binding polymer binders.

For example, Ag/AgCl which the silver containing material comprises, comprises in the range from 20 to 70 wt.-% of Ag, in the range from 20 to 70 wt.-% of AgCl and in the range of 1 to 20 wt.-% of a binder, wherein the wt.-% are in each case based on the sum of the wt.-% of Ag, AgCl and the binder.

If the second layer does not comprise the silver containing material, then a layer of the silver containing material typically is located on the second side of the substrate and covers the second conductive material partially, thereby forming at least one second electrode. For the silver containing material which is not comprised in the second layer the embodiments and preferences described above for the silver containing material which the second layer may comprise apply accordingly.

Thus, the analyte sensor of the invention typically comprises at least one second electrode. Preferably, the first side of the substrate does not comprise a second electrode. It is furthermore preferred that the second electrode does not comprise an enzyme. Thus, preferably the second electrode is free of enzyme. The at least one second electrode may be selected from the group consisting of a counter electrode, a reference electrode and a combined counter/reference electrode. Preferably, the at least one second electrode is a combined counter reference electrode.

A suitable hydrophobic polymer material may be any hydrophobic polymer material known to the skilled person. "Hydrophobic" within the context of the present invention means that the polymer material has a water uptake in the range from 0 to 5 % by weight, in an embodiment a water uptake of less than 2 % by weight, based on the total weight of the hydrophobic polymer material measured after 24 hours according to ASTM D570.

The hydrophobic polymer material preferably comprises a hydrophobic polymer, particularly a thermoplastic hydrophobic polymer. The hydrophobic polymer material may comprises further components, such as binders, solvents and/or crosslinkers.

The hydrophobic polymer, for example, has a glass transition temperature in the range from -100 °C to 0 °C, preferably in the range from -70 °C to -50 °C. The glass transition temperature may be measured via differential scanning calorimetry using a ramp of 10 °C/min for heating and cooling. The glass transition temperature is measured during the second heating cycle. This means that first, the hydrophobic polymer is heated with a ramp of 10 °C/min, then it is cooled with a ramp of 10 °C/min and then it is heated again with a ramp of 10 °C/min to determine the glass transition temperature.

The hydrophobic polymer, for example, has a crystallization temperature in the range from 50 °C to 100 °C, for example in the range from 75 °C to 85 °C. The crystallization temperature is measured via differential scanning calorimetry using the same parameters as for the glass transition temperature.

The hydrophobic polymer may be any hydrophobic polymer known to the skilled person. Preferably, the hydrophobic polymer is selected from the group consisting of thermoplastic polyurethanes (TPU), thermoplastic polyurea, polyethylene, polypropylene, polystyrene, butyl methacrylate polymers (BUMA), polyethylene terephtalate (PET), and UV hardening resins, such as acrylated silicones, acrylated urethanes, acrylated polyesters and/or acrylated epoxides. Preferably, the hydrophobic polymer is a thermoplastic polyurethane.

The hydrophobic thermoplastic polyurethane may comprise hard segments and soft segments in various ratios. Suitable hard segments usually comprise a polymerization product of a diisocyanate and a polyol. A suitable diisocyanate may be an aliphatic diisocyanate or an aromatic diisocyanate, preferably an aliphatic diisocyanate.

Suitable aromatic diisocyanates are for example, 4,4'-methylene diphenyl diisocyanate, and/or toluene-2,4-diisocyanate.

Suitable aliphatic diisocyanates are for example, hexamethylene diisocyanate, and/or isophorone diisocyanate.

A suitable polyol is preferably a diol, such as 1,4-butanediol, 1,5-pentanediol, 1,6-hexanediol, and/or 1,10-decanediol.

Suitable soft segments may comprise polyethers and/or polyesters. Suitable polyethers are for example polyethylene oxide and/or polytetrahydrofurane, whereas suitable polyesters are for example polyethylene terephthalate and/or polyethylene naphthalate.

The first layer has a varying thickness along the length of the substrate and/or is located on the first side of the substrate as at least two fields of the first layer which are separate from one another wherein each of the at least two fields extends over the whole width of the substrate.

In the following a first layer having a varying thickness along the length of the substrate will be described in more detail.

If the first layer has a varying thickness along the length of the substrate, the first layer may comprise at least one region which has a higher thickness than at least one other region of the first layer. For example, the first layer may have a smaller thickness (i.e. the first layer may be thinner) on the ex-vivo portion. Its thickness may then increase towards the in-vivo portion of the substrate so that the region of highest thickness of the first layer may be at the in-vivo portion of the substrate. It is also possible that the first layer has a smaller thickness at the ex-vivo portion and at the intermediate portion. The thickness may then increase at the in-vivo portion and it may decrease again at the in-vivo portion so that a bulge of the first layer is obtained on the in-vivo portion.

It is also possible that the intermediate portion and/or the ex-vivo portion do not comprise the first layer at all. In this embodiment, the first layer may only be located on the in-vivo portion of the analyte sensor. In particular in this embodiment it is further possible that the first layer has an indentation, so that the thickness in the middle of the first layer is smaller than the thickness at the edge of the first layer, wherein the edges are preferably located along the length of the substrate.

The thickness of the first layer is typically measured in a direction which is perpendicular to the length and the width of the substrate, i.e. the direction of the thickness of the substrate.

For example, the thickest region of the first layer may have a thickness which is from 101 % to 500 %, preferably from 150 % to 200 %, of the thickness of the thinnest region of the first layer.

For example, the thickness of the first layer may vary in a range from 0.1 $\mu$m to 200 $\mu$m, preferably in a range from 0.1 $\mu$m to 80 $\mu$m.

Thus, an analyte sensor is preferred in which the thickness of the first layer varies between 0.1 $\mu$m and 200 $\mu$m.

For example, if the first layer is or comprises an insulating material, then the thickness of the first layer may vary in the range from 0.5 $\mu$m to 15 $\mu$m.

For example, if the first layer is or comprises a sensing material, then the thickness of the first layer may vary in the range from 5 $\mu$m to 40 $\mu$m.

For example, if the first layer is or comprises a biocompatibility material, then the thickness of the first layer may vary in the range from 1 $\mu$m to 30 $\mu$m.

For example, if the first layer is or comprises a flux limiting material, then the thickness of the first layer may vary in the range from 1 $\mu$m to 40 $\mu$m.

It is preferred that the thickest region of the first layer is located on the in-vivo portion of the substrate.

Thus, an analyte sensor is preferred wherein the first layer has a varying thickness along the length of the substrate and wherein the thickness of the first layer is higher in the region of the in-vivo portion of the substrate than the thickness of the first layer in the region of the ex-vivo portion.

The thinnest region is in particular located on the ex-vivo portion of the substrate. This is in particular advantageous as in this case the ex-vivo portion and the intermediate portion may be more flexible (i.e. less stiff) and may absorb mechanical stress on the analyte sensor. The in-vivo portion may then be stiffer and, therefore, also more stable.

If the first layer has a varying thickness along the length of the substrate, then it is preferred that the first layer comprises a material selected from the group consisting of an insulating material, a third conductive material, a biocompatibility material and a sensing material. In particular, if the first layer has a varying thickness along the length of the substrate, then the first layer comprises a material selected from the group consisting of an insulating material and a third conductive material.

Thus, an analyte sensor is preferred wherein the first layer has a varying thickness along the length of the substrate and wherein the first layer comprises a material selected from the group consisting of an insulating material, a third conductive material, a biocompatibility material and a flux limiting material.

Additionally or alternatively to having a varying thickness along the length of the substrate, the first layer may be located on the first side of the substrate as at least two fields of the first layer which are separate from one another wherein each of the at least two fields extends over the whole width of the substrate.

In the following, a first layer being located on the first side of the substrate as at least two fields of the first layer which are separate from one another wherein each of the at least two fields extends over the whole width of the substrate will be described in more detail.

To the skilled person it is clear that in this embodiment, the two fields of the first layer are separate from one another along the length of the substrate. Thus, the at least two fields of the first layer form individual fields which may be adjacent to each other but which do not touch each other. Thus, there is a space between the at least two fields along the length of the substrate which is free of the first layer.

For example, the at least two fields of the first layer may be spaced at least 0.1 mm, preferably at least 1 mm distant from each other.

The length of each of the at least two fields of the first layer may be in the range from 0.1 mm to 10 mm, preferably in the range from 0.5 to 5 mm.

In an embodiment, each of the at least two fields of the first layer has the same length. It is also possible that the at least two fields of the first layer have different lengths.

The length of the at least two fields of the first layer is typically measured in the direction of the length of the substrate.

The at least two fields of the first layer may have a thickness in the range from 0.1 $\mu$m to 200 $\mu$m, preferably in the range from 0.1 $\mu$m to 80 $\mu$m.

For example, if the first layer is or comprises an insulating material, then the thickness of the first layer may be in the range from 0.5 $\mu$m to 15 $\mu$m.

For example, if the first layer is or comprises a sensing material, then the thickness of the first layer may be in the range from 5 $\mu$m to 40 $\mu$m.

For example, if the first layer is or comprises a biocompatibility material, then the thickness of the first layer may be in the range from 1 $\mu$m to 30 $\mu$m.

For example, if the first layer is or comprises a flux limiting material, then the thickness of the first layer may be in the range from 1 μm to 40 μm.

The thickness of the at least two fields of the first layer is typically measured in a direction which is perpendicular to the length and the width of the substrate, i.e. in the same direction as the thickness of the substrate.

Preferably, the at least two fields of the first layer are located on the in-vivo portion of the substrate.

In an embodiment, the first layer is located on the first side of the substrate as at least two fields of the first layer and the first layer comprises at least one material selected from the group consisting of an insulating material, a third conductive material and a sensing material.

Thus, an analyte sensor is preferred, wherein the first layer is located on the first side of the substrate as at least two fields, and wherein the first layer comprises at least one material selected from the group consisting of an insulating material, a third conductive material and a sensing material.

The second layer has a varying thickness along the length of the substrate and/or is located on the second side of the substrate as at least two fields of the second layer which are separate from one another wherein each of the at least two fields extends over the whole width of the substrate.

In the following a second layer having a varying thickness along the length of the substrate will be described in more detail.

If the second layer has a varying thickness along the length of the substrate, the second layer may comprise at least one region which has a higher thickness than at least one other region of the second layer. For example, the second layer may have a smaller thickness (i.e. the first layer may be thinner) on the ex-vivo portion. Its thickness may then increase towards the in-vivo portion of the substrate so that the region of highest thickness of the second layer may be at the in-vivo portion of the substrate. It is also possible that the second layer has a smaller thickness at the ex-vivo portion and at the intermediate portion. The thickness may then increase at the in-vivo portion and it may decrease again at the in-vivo portion so that a bulge of the second layer is obtained.

It is also possible that the intermediate portion and/or the ex-vivo portion do not comprise the second layer at all. In this embodiment, the second layer may only be located on the in-vivo portion of the analyte sensor. In particular in this embodiment it is further possible that the second layer has an indentation, so that the thickness in the middle of the second layer is smaller than the thickness at the edge of the second layer, wherein the edges are preferably located along the length of the substrate.

The thickness of the second layer is typically measured in a direction which is perpendicular to the length and the width of the substrate, i.e. the direction of the thickness of the substrate.

For example, the thickest region of the second layer may have a thickness which is from 101 % to 500 %, preferably from 150 % to 200 %, of the thickness of the thinnest region of the second layer. For example, the thickness of the second layer may vary in a range from 0.1 μm to 200 μm, preferably in a range from 0.1 μm to 80 μm.

Thus, an analyte sensor is preferred in which the thickness of the second layer varies between 0.1 μm and 200 μm.

For example, if the second layer is or comprises an insulating material, then the thickness of the second layer may vary in the range from 0.5 μm to 15 μm.

For example, if the second layer is or comprises a biocompatibility material, then the thickness of the second layer may vary in the range from 1 μm to 30 μm.

For example, if the second layer is or comprises a flux limiting material, then the thickness of the second layer may vary in the range from 1 μm to 40 μm.

For example, if the second layer is or comprises a silver containing material, then the thickness of the second layer may vary in the range from 1 μm to 80 μm.

For example, if the second layer is or comprises a hydrophobic polymer material, then the thickness of the second layer may vary in the range from 1 μm to 40 μm.

It is preferred that the thickest region of the second layer is located on the in-vivo portion of the substrate.

Thus, an analyte sensor is preferred wherein the second layer has a varying thickness along the length of the substrate and wherein the thickness of the second layer is higher in the region of the in-vivo portion of the substrate than the thickness of the second layer in the region of the ex-vivo portion.

The varying thickness of the second layer and/or the varying thickness of the first layer along the length of the substrate typically results in a varying stiffness of the analyte sensor along its length.

Thus, an analyte sensor is preferred wherein the analyte sensor has a varying stiffness along its length.

The thinnest region of the first layer and/or of the second layer is in particular located on the ex-vivo portion of the substrate. This is in particular advantageous as in this case the ex-vivo portion and the intermediate portion may be more flexible (i.e. less stiff) and may absorb mechanical stress. The in-vivo portion may then be stiffer and, therefore, also more stable.

Thus, an analyte sensor is preferred wherein the stiffness of the ex-vivo portion is smaller than the stiffness of the in-vivo portion.

If a projection of the thickest region of the first layer into the plane of the substrate and the projection of the thickest region of the second layer into the plane of the substrate overlap (i.e. the thickest region of the first layer and the thickest region of the second layer directly oppose each other), the region in which they overlap is usually the stiffest region of the sensor. The sensor is, thus, more stable and other regions, in particular the ex-vivo region may be more flexible and absorb mechanical stress which is applied to the sensor.

In an embodiment a projection of the thickest region of the first layer into the plane of the substrate and the projection of the thickest region of the second layer into the plane of the substrate do not overlap (i.e. the thickest region of the first layer and the thickest region of the second layer alternate). In this embodiment, the bending direction of the analyte sensor in case of mechanical stress on the analyte sensor may be determined by the arrangement of the thickest regions relative to one another.

If the second layer has a varying thickness along the length of the substrate, then it is preferred that the second layer comprises a material selected from the group consisting of an insulating material, a fourth conductive material, a biocompatibility material, a silver containing material and a hydrophobic polymer material. In particular, if the second layer has a varying thickness along the length of the substrate, then the second layer comprises a material selected from the group consisting of an insulating material, a silver containing material and a fourth conductive material.

Thus, an analyte sensor is preferred wherein the second layer has a varying thickness along the length of the substrate and wherein the second layer comprises a material selected from the group consisting of an insulating material, a fourth conductive material, a biocompatibility material, a silver containing material and a hydrophobic polymer material.

Additionally or alternatively to having a varying thickness along the length of the substrate, the second layer may be located on the second side of the substrate as at least two fields of the second layer which are separate from one another wherein each of the at least two fields extends over the whole width of the substrate.

In the following, a second layer being located on the second side of the substrate as at least two fields of the second layer which are separate from one another wherein each of the at least two fields extends over the whole width of the substrate will be described in more detail.

To the skilled person it is clear that in this embodiment, the two fields of the second layer are separate from one another along the length of the substrate. Thus, the at least two fields of the second layer form individual fields which may be adjacent to each other but which do not touch each other. Thus, there is a space between the at least two fields along the length of the substrate which is free of the second layer.

For example, the at least two fields of the second layer may be spaced at least 0.1 mm, preferably at least 1 mm distant from each other.

The length of each of the at least two fields of the second layer may be in the range from 0.1 mm to 10 mm, preferably in the range from 0.5 mm to 5 mm.

In an embodiment, each of the at least two fields of the second layer has the same length. It is also possible that the at least two fields of the second layer have different lengths.

The length of the at least two fields of the second layer is typically measured in the direction of the length of the substrate.

The at least two fields of the second layer may have a thickness in the range from 0.1 $\mu$m to 200 $\mu$m, preferably in the range from 0.1 $\mu$m to 80 $\mu$m.

For example, if the second layer is or comprises an insulating material, then the thickness of the second layer may be in the range from 0.5 $\mu$m to 15 $\mu$m.

For example, if the second layer is or comprises a biocompatibility material, then the thickness of the second layer may be in the range from 1 $\mu$m to 30 $\mu$m.

For example, if the second layer is or comprises a flux limiting material, then the thickness of the second layer may be in the range from 1 $\mu$m to 40 $\mu$m.

For example, if the second layer is or comprises a silver containing material, then the thickness of the second layer may be in the range from 1 $\mu$m to 80 $\mu$m.

For example, if the second layer is or comprises a hydrophobic polymer material, then the thickness of the second layer may be in the range from 1 $\mu$m to 40 $\mu$m.

The thickness of the at least two fields of the second layer is typically measured in a direction which is perpendicular to the length and the width of the substrate.

Preferably, the at least two fields of the second layer are located on the in-vivo portion of the substrate.

In an embodiment, the second layer is located on the second side of the substrate as at least two fields, wherein the second layer comprises at least one material selected from the group consisting of an insulating material, a fourth conductive material, a silver containing material and a hydrophobic polymer material, preferably selected from the group consisting of an insulating material, a fourth conductive material, and a silver containing material.

Thus, an analyte sensor is preferred, wherein the second layer is located on the second side of the substrate as at least two fields and wherein the second layer comprises at least one material selected from the group consisting of an insulating material, a fourth conductive material, a silver containing material and a hydrophobic material.

In an embodiment, the projection of the at least two fields of the first layer into the plane of the substrate and the projection of the at least two fields of the second layer into the plane of the substrate do not overlap (i.e. the at least two fields of the first layer and the at least two fields of the second layer alternate). In this embodiment, the bending direction of the analyte sensor in case of mechanical stress on the analyte sensor may be determined by the arrangement of the at least two fields of the first layer and of the second layer relative to one another. Furthermore, this arrangement may result in a behavior of the analyte sensor similar to a spring. Thus, the analyte sensor then can reliably compensate compression and/or buckling of the analyte sensor.

Therefore, an analyte sensor is preferred wherein the first layer is located on the first side of the substrate as at least two fields of the first layer and wherein the second layer is located on the second side of the substrate as at least two fields, wherein a projection of the at least two fields of the first layer into the plane of the substrate and a projection of the at least two fields of the second layer into the plane of the substrate do not overlap.

In another embodiment, the projection of the at least two fields of the first layer into the plane of the substrate and the projection of the at least two fields of the second layer into the plane of the substrate overlap (i.e. at least two fields of the first layer and the at least two fields of the second layer directly oppose each other). The region in which the at least two fields of the first layer and of the second layer overlap is usually particularly stiff. Thus, the sensor is more stable and other region, in particular the ex-vivo portion and/or the intermediate portion may be more flexible and absorb mechanical stress which is applied to the sensor.

Another object of the present invention is a method for manufacturing an analyte sensor, in particular an inventive analyte sensor, the method comprising the steps:

   a) providing a raw substrate which has a width and a length and which comprises a first side and a second side opposing the first side and at least one first conductive material which is located on the first side and at least one second conductive material which is located on the second side,
   b) applying a first layer onto the first side of the raw substrate in a manner that it covers the first conductive material partially, and in a manner that

   • it has a varying thickness along the length of the raw substrate and/or
   • it is applied in the form of at least two fields which are separate from one another,

   c) applying a second layer onto the second side of the raw substrate in a manner that it covers the second conductive material partially and in a manner that

   • it has a varying thickness along the length of the raw substrate and/or
   • it is applied in the form of at least two fields which are separate from one another,

   d) cutting the raw substrate to obtain the analyte sensor.

Process steps a) to d) may be carried out in the given order. However, it is also possible to carry out the steps in different orders. In particular, the order of steps b) and c) may be different. Further process steps are feasible. It is also possible to carry out at least one of process steps a) to d) more than once. For example, steps b) and/or c) may be carried out more than once so that more than one first layer and/or more than one second layer is applied.

In particular, further process steps may be carried out to apply a sensing material in case the first layer does not comprise a sensing material.

In particular further process steps may be carried out to apply a silver containing material in case the second layer do not comprise a silver containing material.

In particular further process steps may be carried out to apply a flux limiting material in case the first layer does not comprise a flux limiting material.

In step a) of the method for manufacturing an analyte sensor, a raw substrate is provided.

Within the context of the present invention, the term "raw substrate" specifically may refer without limitation to any kind of material or combination of materials which is suitable to form a carrier layer to support the first layer and the second layer.

From the raw substrate, the substrate of the inventive analyte sensor is manufactured by cutting the raw substrate. In particular, the raw substrate may comprise an electrically insulating material. For the electrically insulating material, the embodiments and preferences described above for the electrically insulating material of the substrate hold true.

Thus in a preferred embodiment the raw substrate comprises at least one electrically insulating material selected from the group consisting of an insulating epoxy resin, a polycarbonate, a polyester, a polyvinylchloride, a polyurethane, a polyether, a polyethylene, a polyamide, a polyimide, a polyacrylate, a polymethacrylate, a polytetrafluoroethylene or a copolymer thereof, and alumina.

A suitable polyester is for example polyethyleneterephthalate.

However, a length and a width of the raw substrate may, in general, considerably differ from the length and the width of the substrate as comprised by the completed analyte sensor. The length and the width of the raw substrate within the context of the present invention are defined with reference to the width and the length of the substrate of the analyte sensor. Thus, the direction of the length of the raw substrate within the context of the present invention corresponds to the direction of the length of the substrate of the analyte sensor which is cut from the raw substrate. Correspondingly, the direction of the width of the raw substrate within the context of the present invention corresponds to the direction of the width of the substrate of the analyte sensor which is cut from the raw substrate.

Preferably, the raw substrate may have a width of 1 cm to 2 km, more preferred of 10 cm to 1000 m, and a length of 1 cm to 100 cm, more preferred of 2 cm to 10 cm. In a preferred embodiment, the raw substrate can be provided as a roll, in particular designated for being used in a roll-to-roll process.

The raw substrate comprises at least one first conductive material and at least one second conductive material. For the at least one first conductive material and the at least one second conductive material the embodiments and preferences as described above for the first conductive material and the second conductive material of the analyte sensor apply.

Providing the raw substrate in step a) may comprise the following steps:

a1) providing a raw substrate,
a2) applying at least one first conductive material to a first side of the raw substrate,
a3) applying at least one second conductive material to a second side of the raw substrate,
a4) obtaining the raw substrate which comprises at least one first conductive material and at least one second conductive material.

The first conductive material can be applied to the raw substrate in step a2) by any known method, for example via chemical vapor deposition (CVD), physical vapor deposition (PVD), or a wet-coating process. Wet-coating processes are known as such. A suitable wet-coating process is for example selected from the group consisting of spin-coating, spray-coating, doctor-blading, printing, dispensing, slot-coating, dip coating and screen printing.

The second conductive material can be applied to the raw substrate in step a3) by any known method, for example via chemical vapor deposition (CVD), physical vapor deposition (PVD), or a wet-coating process. Wet-coating processes are known as such. A suitable wet-coating process is for example selected from the group consisting of spin-coating, spray-coating, doctor-blading, printing, dispensing, slot-coating, dip coating and screen printing.

The raw substrate comprises a first side and a second side. The second side opposes the first side. To the person skilled in the art it is clear that the first side and the second side are different from one another.

The raw substrate may be a flat substrate. Specifically the raw substrate may be flexible and/or deformable. Thus, as an example, the raw substrate may be a thin, flexible raw substrate. As an example, the raw substrate may have a thickness of 50 $\mu$m to 1 mm, specifically a thickness of 80 $\mu$m to 500 $\mu$m, such as 110 $\mu$m to 250 $\mu$m.

The raw substrate may be provided by any method known to the skilled person. For example, the raw substrate may be provided as a roll. This is particularly advantageous as the raw substrate may then be used in a roll-to-roll process.

In an embodiment, the raw substrate is cut into sheets before the first electrode is prepared. The sheets may have any width, such as, for example, in the range from 100 mm to 300 mm.

In step b) a first layer is applied onto the first side of the raw substrate in a manner that it covers the first conductive material partially.

For the first layer applied in the method the embodiments and preferences as described above for the first layer comprised in the analyte sensor apply accordingly.

The first layer may be applied onto the first side by any method known to the skilled person, for example by a wet-coating process. A suitable wet-coating process is for example selected from the group consisting of spin-coating, spray-coating, doctor-blading, printing, dispensing, slot-coating, dip coating and screen printing. More than one first layer may be applied which may result in a varying thickness along the length of the substrate. After the wet-coating process, the first layer may be further treated. Such treatments are for example drying treatments, curing treatments and/or laser ablation treatments. In particular, laser ablation treatments may be used to form at least two fields of the first layer which are separate from one another. However, it is also possible that during the wet-coating process the separate fields are formed.

In step c) a second layer is applied onto the second side of the substrate in a manner that it covers the second conductive material partially.

For the second layer applied in the method the embodiments and preferences as described above for the second layer comprised in the analyte sensor accordingly.

The second layer may be applied onto the second side by any method known to the skilled person, for example by a wet-coating process. A suitable wet-coating process is for example selected from the group consisting of spin-coating, spray-coating, doctor-blading, printing, dispensing, slot-coating, dip coating and screen printing. More than one second layer may be applied which may result in a varying thickness along the length of the substrate. After the wet-coating process, the first layer may be further treated. Such treatments are for example drying treatments, curing treatments, and/or laser ablation treatments. In particular, laser ablation treatments may be used to form at least two fields of the second layer which are separate from one another. However, it is also possible that during the wet-coating process the separate fields are formed.

In step d) the raw substrate is cut to obtain the analyte sensor. The raw substrate is preferably cut along its length so that strips are formed. These strips may correspond to the analyte sensor. It is possible that before or after the saw substrate is cut along its length it is cut at least once along its width.

The raw substrate may be cut by any method known to the skilled person. For example, it may be cut by mechanical methods such as by a knife and/or a saw. It is also possible to cut the raw substrate with a laser.

This embodiment is preferred.

Thus a method for producing an analyte sensor is preferred, wherein in step d) the raw substrate is cut using a laser beam.

In an embodiment, the following additional step e) may be carried out:

e) electronically connecting the analyte sensor with an electronics unit to obtain an analyte sensor system comprising the analyte sensor and the electronics unit.

A further object of the present invention is an analyte sensor system comprising:

- the inventive analyte sensor and
- an electronics unit, the electronics unit being in electronically connected to the analyte sensor.

For the analyte sensor comprised in the analyte sensor system, the embodiments and preferences described above for the inventive analyte sensor hold true.

The term "electronics unit" as used herein is a broad term and is to be given its ordinary and customary meaning to a person of ordinary skill in the art and is not to be limited to a special or customized meaning. The term specifically may refer, without limitation, to a unit, such as a unit which may be handled as a single piece, which is configured for performing at least one electronic function. Specifically, the electronics unit may have at least one interface for being connected to the analyte sensor, wherein the electronics unit may provide at least one electronic function interacting with the analyte sensor, such as at least one measurement function. The electronics unit specifically may be configured for measuring at least one voltage and/or for measuring at least one current, thereby interacting with the analyte sensor. The electronics unit may further comprise at least one integrated circuit, such as a processor and/or a battery. The term "processor" as generally used herein is a broad term and is to be given its ordinary and customary meaning to a person of ordinary skill in the art and is not to be limited to a special or customized meaning. The term specifically may refer, without limitation, to an arbitrary logic circuitry configured for performing basic operations of a computer or system, and/or, generally, to a device which is configured for performing calculations or logic operations. In particular, the processor may be configured for processing an electronic signal, such as a current or a voltage, specifically an electronic signal from the analyte sensor. Specifically, the processor may be or may comprise a microcontroller unit (MCU). Additionally or alternatively, the processor may be or may comprise a microprocessor, thus specifically the processor's elements may be contained in one single integrated circuitry (IC) chip. Additionally or alternatively, the processor may be or may comprise one or more application-specific integrated circuits (ASICs) and/or one or more field-programmable gate arrays (FPGAs) or the like. The processor specifically may be configured, such as by software programming, for performing one or more evaluation operations. Thus, the processor may be configured for processing and/or evaluating the electronic signal from the analyte sensor and, for example, outputting a signal indicating the analyte concentration measured by the analyte sensor. The electronics unit further may comprise at least one measuring device for measuring at least one of a voltage and a current, such as a potentiostat. Further, the electronics unit may comprise a microcontroller, specifically being configured for controlling one or more electronic functions of the electronics unit.

The electronics unit specifically may comprise at least one electronics unit housing, wherein the analyte sensor, e.g. with a proximal end and/or an end providing electrical contacts for contacting the analyte sensor, may protrude into the electronics unit housing and may be electrically connected with at least one electronic component within the electronics unit housing. As an example, the proximal end and/or at least one contact portion of the analyte sensor may protrude into the electronics unit housing and, therein, may be electrically connected to at least one electronic component, such as to at least one printed circuit board and/or at least one contact portion of the electronics unit, e.g. by one or more of a soldering connection, a bonding connection, a plug, a clamping connection or the like. The electronics unit specifically may be used and/or configured as a transmitter for transmitting measurement data to at least one external device, such as to at least one receiver, e.g. wirelessly.

The electronics unit is electronically connected to the analyte sensor. Thus, an electrical connection exits between the analyte sensor and the electronics unit. The electronics unit comprised in the analyte sensor system is in contact with the analyte sensor. For example, the first conductive material and the second conductive material of the analyte sensor may each form an electrical connection with the electronics unit. Typically, the analyte sensor comprises the contact portion at a proximal end and the first electrode and the second electrode at a distal end. Thus, an electrical signal, such as an electrical current and/or an electric voltage, may be transmitted via the electronic connection from the analyte sensor to the electronics unit. Via the electrical connection, the electronics unit may interact with the analyte sensor for performing at least one electrochemical measurement. The electrical connection specifically, as outlined above, may be established by at least one connection portion of the analyte sensor protruding into a housing of the electronics unit.

Short description of the figures

Further details of the invention can be derived from the following disclosure of preferred embodiments. The features of the embodiments can be implemented in an isolated way or in any combination. The invention is not restricted to the embodiments. The embodiments are schematically depicted in the Figures. The Figures are not to scale. Identical reference numbers in the Figures refer to identical elements or functionally identical elements or elements corresponding to each other with regard to their functions.

In the figures:

Figure 1: schematically illustrates an aerial view of a first embodiment of an analyte sensor according to the present invention

Figures 2a-2c: schematically illustrate different views of a second embodiment of an analyte sensor according to the present invention

Figures 3a-3c: schematically illustrate different views of a third embodiment of an analyte sensor according to the present invention

Figures 4a-4c: schematically illustrate different views of a fourth embodiment of an analyte sensor according to the present invention

Detailed description of the embodiments

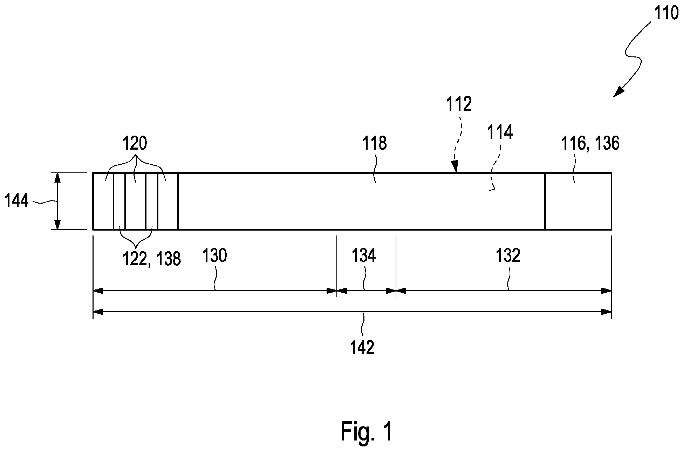

In the figures, no flux limiting material, biocompatibility material or hydrophobic polymer material are shown. To the skilled person it is clear that those materials may additionally cover the analyte sensor as described elsewhere herein. It is furthermore emphasized that the dimensions used in any of Figures 1 to 4c are not to scale. The embodiments and preferences as described above for the substrate, the first conductive material, the second conductive material, the first layer and the second layer apply also for the substrate, the first conductive material, the second conductive material, the first layer and the second layer described with respect to the figures.

Figure 1 shows an analyte sensor 110 according to a first embodiment of the present invention. In Figure 1 only the first side 114 of the substrate 112 is shown. The second side 124 opposing the first side is not shown in Figure 1. The analyte sensor 110 comprises a substrate 112 which comprises a first conductive material 116 located on the first side 114 of the substrate 112. The first conductive material 116 is partially covered by an insulating material 118, such as a solder mask. The analyte sensor 110 furthermore comprises a third conductive material 120. The third conductive material 120 forms a layer having a constant thickness over the length of the third conductive material 120. Two fields of a first layer 138 which comprises a sensing material 122 are located on top of the third conductive material 120. The analyte sensor 110 comprises an electrical contact 136 comprising the first conductive material 116 for contacting the analyte sensor 110 with an electronics unit such as a printed circuit board (PCB). The electrical contact 136 is located on the ex-vivo portion 132 of the analyte sensor 110. The third conductive material 120 as well as the two fields of the first layer 138 are located on the in-vivo portion 130 of the analyte sensor 110. The intermediate portion 134 connects the in-vivo portion 130 with the ex-vivo portion 132. The substrate 112 of the analyte sensor 110 has a width 144 and a length 142.

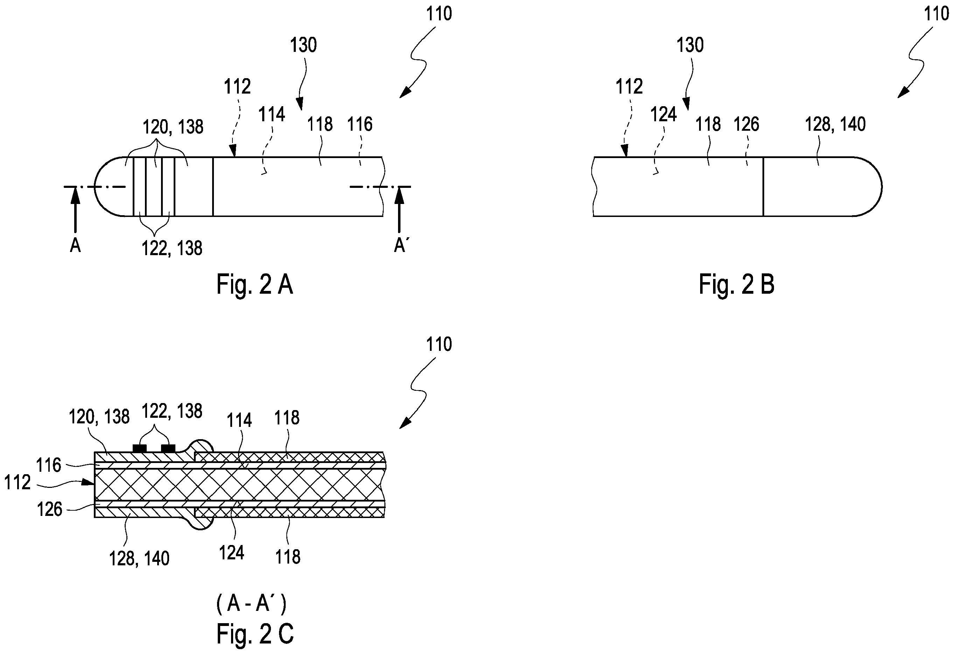

Figures 2a, 2b and 2c show an analyte sensor 110 according to a second embodiment of the present invention. In Figures 2a, 2b and 2c only the in vivo portion of the analyte sensor 110 is shown. The waved lines indicate where the part of the analyte sensor which is not shown, in particular the ex-vivo portion and the intermediate portion, is located. Figure 2a shows a view of the first side 114 of the substrate 112, Figure 2b shows a view of the second side 124 of the substrate 112, Figure 2c shows a traverse section along the length of the analyte sensor 110 (along line A-A' in Figure 2a). In Figures 2a to 2c the analyte sensor 110 comprises a substrate 112.

On the first side 114 of the substrate 112 a first conductive material 116 is located. The first conductive material 116 is partially covered by an insulating material 118. A first layer 138 is located on the first side 114 of the substrate 112 and covers it partially. It also covers the insulating material 118 partially. The first layer 138 comprises a third conductive material 120. The first layer 138 comprising the third conductive material 120 has a varying thickness along the length of the substrate 112. The analyte sensor 110 furthermore comprises a first layer 138 comprising a sensing material 122 located on the first side 114 of the substrate 112 on top of the first layer 138 comprising the third conductive material 120. The first layer 138 comprising the sensing material 122 has two fields separate from one another which extend over the whole width of the substrate 112.

On the second side 124 of the substrate 112 a second conductive material 126 is located. The second conductive material 126 is partially covered by an insulating material 118 which may be the same as or different from the insulating material 118 located on the first side 114 of the substrate 112. A second layer 140 is located on the second side 124 of the substrate 112 and covers it partially. It also covers the insulating material 118 partially. The second layer 140 comprises a silver containing material 128. The second layer 140 comprising the silver containing material 128 has a varying thickness along the length of the substrate 112.

In the analyte sensor 110 shown in Figures 2a to 2c, the thicker regions of the first layer 138 and the second layer 140 oppose each other. This results in an analyte sensor 110 which is particularly stiff in the region in which the first layer 138 and the second layer 140 oppose each other. Thus, the in-vivo portion is stiffer than the intermediate portion and the ex-vivo portion. Any mechanical stress is therefore in particular absorbed by the intermediate portion and by the ex-vivo portion of the analyte sensor 110. This reduces the risk of artifacts during measurement due to mechanical load on the analyte sensor 110, in particular on the in vivo-portion of the analyte sensor 110. Also the risk of damages of the electrodes is significantly reduced.

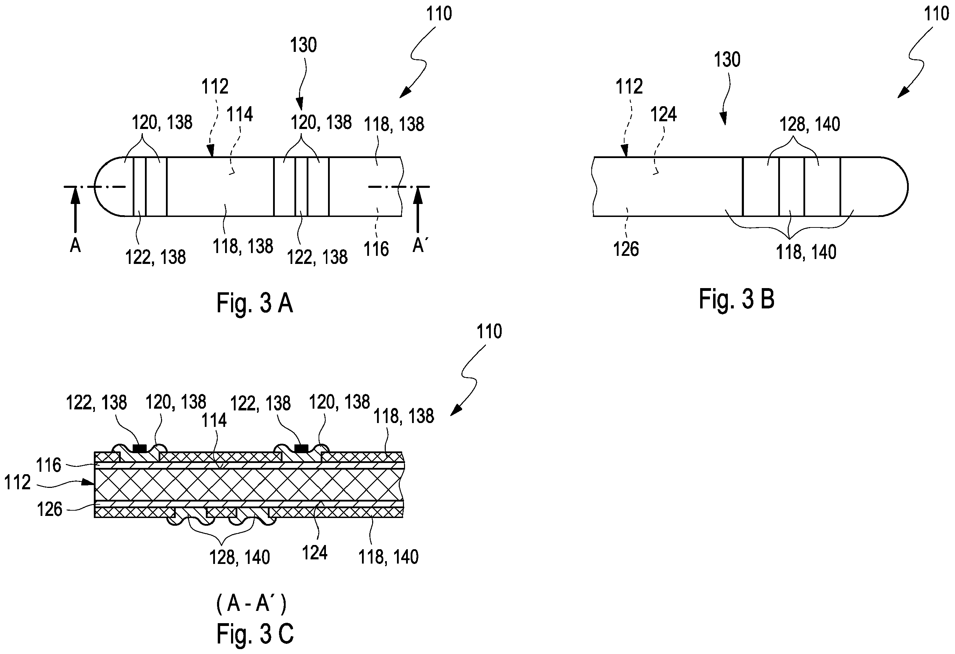

Figures 3a, 3b and 3c show an analyte sensor 110 according to a third embodiment of the present invention. In Figures 3a, 3b and 3c only the in vivo portion of the analyte sensor 110 is shown. The waved lines indicate where the part of the analyte sensor which is not shown, in particular the ex-vivo portion and the intermediate portion, is located. Figure 3a shows a view of the first side 114 of the substrate 112, Figure 3b shows a view of the second side 124 of the substrate 112, Figure 3c shows a traverse section along the length of the analyte sensor 110 (along line A-A' in Figures 3a and 3b). In Figures 3a, 3b and 3c, the analyte sensor 110 comprises a substrate 112.

On the first side 114 of the substrate 112 a first conductive material 116 is located. The first conductive material 116 is partially covered by a first layer 138 comprising an insulating material 118 and forming two fields separate from one another which extend over the whole width of the substrate 112. The analyte sesnsor 110 furthermore comprises a first layer 138 which comprises a third conductive material 120 located on the first side 114 of the substrate 112 and covering the first side 114 as well as the first layer 138 comprising the insulating material 118 partially. The first layer 138 comprising the third conductive material 120 comprises two fields separate from one another which extend over the whole width of the substrate 112. Furthermore, the first layer 138 comprising the third conductive material 120 has a varying thickness along the length of the substrate 112. The analyte sensor 110 additionally comprises a first layer 138 comprising a sensing material 122 located on the first side 114 of the substrate 112 on top of the first layer 138 comprising the third conductive material 120. The first layer 138 comprising the sensing material 122 has two fields separate from one another which extend over the whole width of the substrate 112.

On the second side 124 of the substrate 112 a second conductive material 126 is located. The second conductive material 126 is partially covered by a second layer 140 comprising an insulating material 118. The insulating material 118 may be the same as or different than the insulating material 118 located on the first side 114 of the substrate 112. The second layer 140 comprising the insulating material 118 forms three fields separate from one another which extend over the whole width of the substrate 112. A second layer 140 comprising a silver containing material 128 is located on the second side 124 of the substrate 112 and covers it partially. It also covers the second layer 140 comprising an insulating material 118 partially. The second layer 140 comprising a silver containing material 128 has a varying thickness along the length of the substrate 112. It furthermore comprises two fields separate from one another which extend over the whole width of the substrate. In particular a projection of the second layer 140 comprising the silver containing material 128 into the plane of the substrate 112 and a projection of the first layer 138 comprising the third conductive material 120 into the plane of the substrate 112 do not overlap. The analyte sensor 110 shown in Figures 3a to 3c is in particular advantageous as regions which only comprise the first layer 138 comprising the insulating material 118 and the second layer 140 comprising the insulating material 118 are particularly flexible whereas the other regions are stiffer. Thus, the in-vivo portion of the analyte sensor 110 has a behavior similar to a spring so that it can reliably compensate compression and/or buckling of the analyte sensor 110, in particular of the in-vivo portion of the analyte sensor 110. Due to the arrangement of the first layer 138 comprising the third conductive material 120 relative to the second layer 140 comprising the silver containing material 128, it is furthermore possible to determine the direction into which the analyte sensor 110 shall be bent in case of mechanical stress.

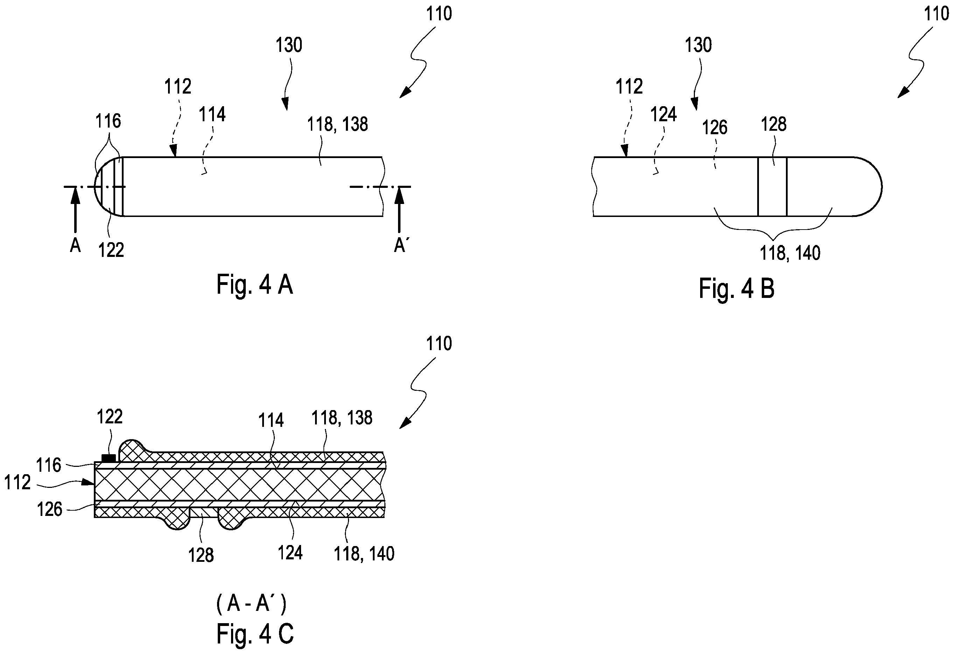

Figures 4a, 4b and 4c show an analyte sensor 110 according to a fourth embodiment of the present invention. In Figures 4a, 4b and 4c only the in vivo portion of the analyte sensor 110 is shown. The waved lines indicate where the part of the analyte sensor which is not shown, in particular the ex-vivo portion and the intermediate portion, is located. Figure 4a shows a view of the first side 114 of the substrate 112, Figure 4b shows a view of the second side 124 of the substrate 112, Figure 4c shows a traverse section along the length of the analyte sensor 110 (along line A-A' in Figures 4a and 4b). In Figures 4a, 4b and 4c, the analyte sensor 110 comprises a substrate 112.

On the first side 114 of the substrate 112 a first conductive material 116 is located. The first conductive material 116 is partially covered by a first layer 138 comprising an insulating material 118. The first layer 138 comprising the insulating material 118 has a varying thickness along the length of the substrate 112. A sensing material 122 is located on the first conductive material 116.

On the second side 124 of the substrate 112 a second conductive material 126 is located. The second conductive material 126 is partially covered by a second layer 140 comprising an insulating material 118. The insulating material 118 may be the same as or different fromsag the insulating material 118 located on the first side 114 of the substrate 112. The second layer 140 comprising the insulating material 118 forms two fields separate from one another which extend over the whole width of the substrate 112. Both fields have a varying thickness along the length of the substrate 112. The second side 124 of the substrate 112 additionally comprises a silver containing material 128 which partially covers the second conductive material 126. In particular, the projection of the thicker regions of the second layer 140 into the plane of the substrate 112 does not overlap with the projection of the thicker region of the first layer 138 into the plane of the substrate 112. This results in an analyte sensor in which the direction of the bending of the in-vivo portion of the analyte sensor can be precisely adjusted. At the same time, a bending of the electrode regions can be avoided by this design.

List of reference numbers

[0227]

| | |
|---|---|
| 110 | analyte sensor |
| 112 | substrate |
| 114 | first side |
| 116 | first conductive material |
| 118 | insulating material |
| 120 | third conductive material |
| 122 | sensing material |
| 124 | second side |
| 126 | second conductive material |
| 128 | silver containing material |
| 130 | in-vivo portion |
| 132 | ex-vivo portion |
| 134 | intermediate portion |
| 136 | electrical contact |
| 138 | first layer |
| 140 | second layer |
| 142 | length |
| 144 | width |

**Claims**

1. An analyte sensor for determining at least one analyte, the analyte sensor comprising:

   - a substrate which has a length and a width and which comprises

      • a first side and a second side which opposes the first side,
      • an in-vivo portion, an ex-vivo portion and an intermediate portion wherein the intermediate portion connects the in-vivo portion with the ex-vivo portion,

   - at least one first conductive material which is located on the first side of the substrate,
   - at least one second conductive material which is located on the second side of the substrate,
   - at least one first layer which is located on the first side of the substrate and covers the first conductive material partially and

      • which has a varying thickness along the length of the substrate and/or
      • which is located on the first side of the substrate as at least two fields of the first layer which are separate from one another wherein each of the at least two fields extends over the whole width of the substrate,

   - at least one second layer which is located on the second side of the substrate and covers the second conductive material partially and

      • which has a varying thickness along the length of the substrate and/or
      • which is located on the second side of the substrate as at least two fields of the second layer which are separate from one another wherein each of the at least two fields extends over the whole width of the substrate.

2. The analyte sensor according to claim 1, wherein the first layer comprises at least one material selected from the group consisting of a third conductive material, an insulating material, a sensing material, a biocompatibility material and a flux limiting material.

3. The analyte sensor according to claim 1 or 2, wherein the second layer comprises at least one material selected from the group consisting of a fourth conductive material, an insulating material, a silver containing material, a biocompatibility material, a flux limiting material and a hydrophobic polymer material.

4. The analyte sensor according to any one of claims 1 to 3, wherein the first layer is located on the first side of the substrate as at least two fields of the first layer and wherein the second layer is located on the second side of the substrate as at least two fields, wherein a projection of the at least two fields of the first layer into the plane of the substrate and a projection of the at least two fields of the second layer into the plane of the substrate do not overlap.

5. The analyte sensor according to any one of claims 1 to 4, wherein the first layer has a varying thickness along the length of the substrate, and wherein the first layer comprises a material selected from the group consisting of an insulating material, a third conductive material, a biocompatibility material and a flux limiting material.

6. The analyte sensor according to any one of claims 1 to 5, wherein the first layer is located on the first side of the substrate as at least two fields, and wherein the first layer comprises at least one material selected from the group consisting of an insulating material, a third conductive material and a sensing material.

7. The analyte sensor according to any one of claims 1 to 6, wherein the second layer has a varying thickness along the length of the substrate and wherein the second layer comprises at least one material selected from the group consisting of an insulating material, a fourth conductive material, a biocompatibility material, a silver containing material and a hydrophobic polymer material.

8. The analyte sensor according to any one of claims 1 to 7, wherein the second layer is located on the second side of the substrate as at least two fields, and wherein the second layer comprises at least one material selected from the group consisting of an insulating material, a fourth conductive material, a silver containing material and a hydrophobic polymer material.

9. The analyte sensor according to any one of claims 1 to 8, wherein the analyte sensor has a varying stiffness along its length.

10. The analyte sensor according to claim 9, wherein the stiffness of the ex-vivo portion is smaller than the stiffness of the in-vivo portion.

11. The analyte sensor according to any one of claims 1 to 10, wherein the first layer has a varying thickness along the length of the substrate and wherein the thickness of the first layer is higher in the region of the in-vivo portion of the substrate than the thickness of the first layer in the region of the ex-vivo portion.

12. The analyte sensor according to any one of claims 1 to 11, wherein the thickness of the first layer varies between 0.1 $\mu$m and 200 $\mu$m and/or the thickness of the second layer varies between 0.1 $\mu$m and 200 $\mu$m.

13. The analyte sensor according to any one of claims 1 to 12, wherein the second layer has a varying thickness along the length of the substrate and wherein the thickness of the second layer is higher in the region of the in vivo portion of the substrate than in the thickness of the second layer in the region of the ex-vivo portion.

14. A method for manufacturing an analyte sensor according to any one of claims 1 to 13, the method comprising the steps:

   a) providing a raw substrate which has a width and a length and which comprises a first side and a second side opposing the first side and at least one first conductive material which is located on the first side and at least one second conductive material which is located on the second side,
   b) applying a first layer onto the first side of the raw substrate in a manner that it covers the first conductive material partially, and in a manner that

   • it has a varying thickness along the length of the raw substrate and/or
   • it is applied in the form of at least two fields which are separate from one another,

c) applying a second layer onto the second side of the raw substrate in a manner that it covers the second conductive material partially and in a manner that

   • it has a varying thickness along the length of the raw substrate and/or
   • it is applied in the form of at least two fields which are separate from one another,

d) cutting the raw substrate to obtain the analyte sensor.

15. An analyte sensor system comprising

   - an analyte sensor according to any one of claims 1 to 13,
   - an electronics unit, the electronics unit being configured to electronically connect to the analyte sensor.

Fig. 1

Fig. 2 B

Fig. 2 A

(A - A´)
Fig. 2 C

Fig. 3 A

Fig. 3 B

( A - A´ )

Fig. 3 C

EP 4 151 151 A1

Fig. 4 B

Fig. 4 A

( A - A´ )
Fig. 4 C

Europäisches
Patentamt

European
Patent Office

Office européen
des brevets

# EUROPEAN SEARCH REPORT

**Application Number**

**EP 21 19 7921**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/219885 A1 (WANG YI [US] ET AL) 22 July 2021 (2021-07-22) * paragraphs [0062], [0066], [0071]; figures 1,4A,5A,5B,5D * ----- | 1-15 | INV. A61B5/1473 ADD. A61B5/1486 |
| X | CN 109 406 589 A (UNIV ZHEJIANG; ZTE ICT TECHNOLOGIES CO LTD) 1 March 2019 (2019-03-01) * paragraphs [0034] - [0041]; figures 2-4 * ----- | 1-15 | |
| X | WO 2012/158202 A2 (ABBOTT DIABETES CARE INC [US]; STAFFORD GARY A [US]; TAUB MARC B [US]) 22 November 2012 (2012-11-22) * paragraphs [0055] - [0056]; figures 5A-5C * ----- | 1-15 | |

**TECHNICAL FIELDS SEARCHED (IPC)**

A61B

The present search report has been drawn up for all claims

| Place of search | Date of completion of the search | Examiner |
|---|---|---|
| The Hague | 1 February 2022 | Mecking, Nikolai |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
...........................................................................................................................
& : member of the same patent family, corresponding document

EPO FORM 1503 03.82 (P04C01)

**ANNEX TO THE EUROPEAN SEARCH REPORT
ON EUROPEAN PATENT APPLICATION NO.**                  EP 21 19 7921

This annex lists the patent family members relating to the patent documents cited in the above-mentioned European search report.
The members are as contained in the European Patent Office EDP file on
The European Patent Office is in no way liable for these particulars which are merely given for the purpose of information.

01-02-2022

| Patent document cited in search report | | Publication date | Patent family member(s) | | Publication date |
|---|---|---|---|---|---|
| US 2021219885 | A1 | 22-07-2021 | US | 2021219885 A1 | 22-07-2021 |
|  |  |  | WO | 2021138473 A1 | 08-07-2021 |
| CN 109406589 | A | 01-03-2019 | NONE | | |
| WO 2012158202 | A2 | 22-11-2012 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9895091 B2
- WO 2006017359 A
- WO 0136660 A2
- WO 2019166394 A1
- EP 2697388 A1
- WO 2007071562 A1
- WO 2005078424 A1

**Non-patent literature cited in the description**

- **FELDMANN et al.** *Diabetes Technology & Therapeutics,* 2003, vol. 5 (5), 769-779